# EUROPEAN PATENT SPECIFICATION

(11) **EP 0 350 292 B1**
(45) Date of publication and mention of the grant of the patent: **04.05.1994**
(21) Application number: 89306834.6
(22) Date of filing: 05.07.1989
(51) Int. Cl.: C07H 19/173

(54) **Process for preparing 2'-deoxy-beta-adenosine**
Verfahren zur Herstellung von 2'-Deoxy-beta-adenosin
Procédé pour la préparation de 2'-déoxy-bêta-adénosine

(30) Priority: 05.07.1988 JP 166063/88
(43) Date of publication of application: 10.01.1990
(73) Proprietor: Japan Tobacco Inc., Minato-Ku Tokyo 105 (JP); YUKI GOSEI KOGYO CO., LTD., Chiyoda-ku Tokyo 102 (JP)
(72) Inventor: Kawakami, Hiroshi, c/o Life Science Research Lab., Yokohama-shi, Kanagawa 227 (JP); Matsushita, Hajime, c/o Life Science Research Lab., Yokohama-shi, Kanagawa 227 (JP); Yoshikoshi, Hajime, c/o Tokyo Lab., Itabashi-ku, Tokyo 174 (JP); Itoh, Kazuo, c/o Tokyo Lab., Itabashi-ku, Tokyo 174 (JP); Naoi, Yoshitake, c/o Tokyo Lab., Itabashi-ku, Tokyo 174 (JP)
(74) Representative: Holmes, Michael John

(56) References cited:
- EP-A- 0 173 059
- JOURNAL OF THE AMERICAN CHEMICAL SOCIETY, vol. 106, 1984, American Chemical Society; Z. KAZIMIERCZUK et al., pp. 6379-6382#
- CHEMICAL ABSTRACTS, vol. 90, no. 11, 12 March 1979, Columbus, OH (US); A. HOLY, p. 658, no. 87785q#
- JOURNAL OF THE AMERICAN CHEMICAL SOCIETY, vol.105, no. 12, 15 June1983, American Chemical Society; M.J. ROBBINS et al., pp. 4059-4065#
- CHEMICAL ABSTRACTS, vol. 98, no. 17, 25 April 1983, Columbus, OH (US); H. GRIENGL et al., p. 617, no. 143787w#

## Description

The present invention relates to a process for preparing 2′-deoxy-β-adenosine having the following formula [I]: 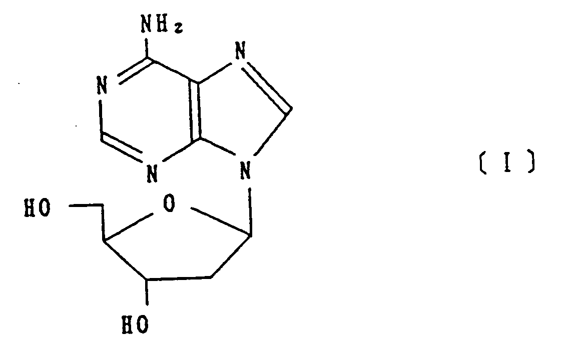
2′-deoxy-β-adenosine obtained by the present invention is a starting material for a compound which is useful as a medicine, and it is a constituent of deoxyribonucleic acid. Consequently demand for 2′-deoxy-β-adenosine is increasing with the advance of genetic engineering in recent years.

Conventionally 2′-deoxy-β-adenosine is prepared from natural deoxyribonucleic acid by decomposing it with enzymes. However, such process is not suitable for industrial production because the starting material is restricted in availability.

The following processes of chemical synthesis are known:
(1) Derivatives of adenine are allowed to condense with derivatives of 2-deoxy-D-erythro-pentofuranose to obtain an equivalent mixture of anomers and then the β-anomer is separated. [Biochimica et Biophysica Acta, 145, 221 (1967)].
(2) The 2-position hydroxyl group of adenosine is eliminated to obtain 2′-deoxy-β-adenosine. [Journal of American Chemical Society, 105, 4059 (1983); Journal of Organic Chemistry, 47, 485 (1982)].

In the process of the above (1), it is complicated to separate β-type compound desired from a mixture of anomers, and in the process of the above (2), poisonous tin compounds have to be used. Therefore these processes are not suitable for industrial use.

Recently, a synthesis of 2′-deoxy-β-adenosine has been reported whereby a sodium salt of a 6-chloropurine derivative and 1-chloro-2-deoxy-3,5-di-O-p-toluoyl-α-D-erythro-pentofuranose are subjected to β-selective condensation. [Journal of American Chemical Society, 106, 6379 (1984); TOKKYO-KOKAI-KOHO (18 month Publication of Unexamined Patent Application) SHOWA 61 (1986)-1065941].

In this process, there are the disadvantages that (1) the 6-chloropurine derivative used as a starting material is expensive; (2) sodium hydride used in the preparation of the sodium salt is dangerous; (3) in the substitution of the chlorine atom by an amino group, heating and pressurization are required.

Consequently, the above conventional proceses are not suitable for industrial use.

The inventors investigated a process for preparing selectively and efficiently β-type isomers having the formula [I] as mentioned above, and found that compound [I] can be obtained by allowing Compound [III] to condense with compound [IV] without a catalyst to obtain the required β-isomer with a selectivity of 90% or more, and then eliminating the protecting groups of purified Compound [II].

Namely, the present invention relates to a process for preparing 2′-deoxy-β-adenosine characterized in that a salt of adenine having the general formula [IV]: 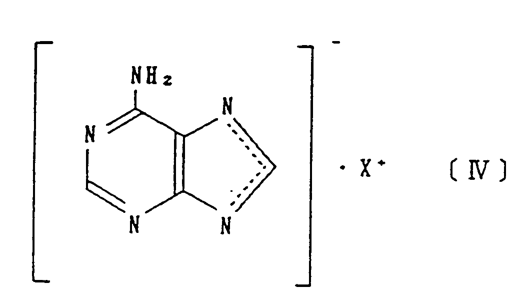
(wherein X⁺ is a cation)
is allowed to condense with a derivative of 1-chloro-2-deoxy-α-D-erythro-pentofuranose having the general formula [III]: 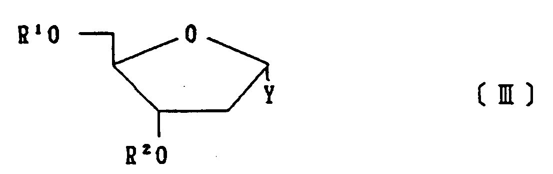
(wherein R¹ and R² are independently hydroxyl protecting groups and Y is a halogen)
to yield a protected derivative of 9-(2-deoxy-β-D-erythropentofuranosyl) adenine having the general formula [II]: 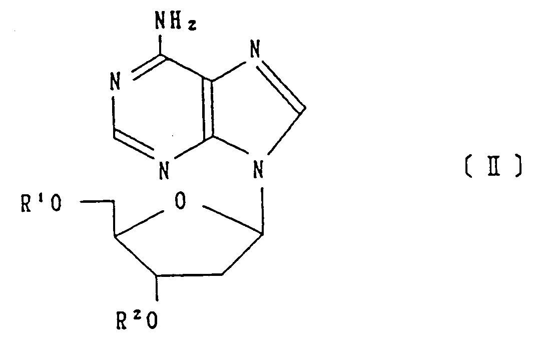
(wherein R¹ and R² are as above defined)
and then the protecting groups are eliminated to yield 2′-deoxy-β-adenosine having the formula [I]: 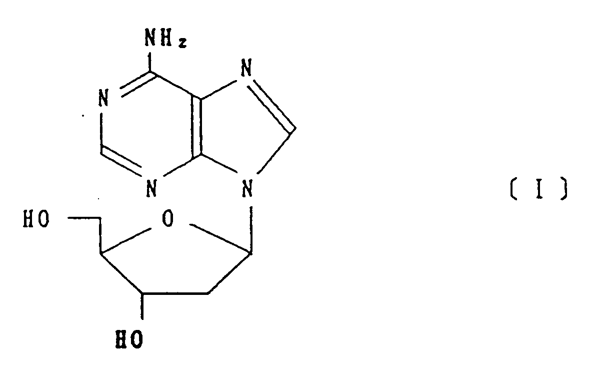
For protecting the two hydroxyl groups of Compound [III], the following groups, which are used as protecting groups for saccharides, may advantageously be employed; aralkyl groups such as the benzyl and trityl groups; acyl groups such as the acetyl, propionyl, pivaloyl and benzoyl groups; alkyloxy carbonyl groups such as the ethoxy-carbonyl group; aryloxycarbonyl groups such as the phenoxycarbonyl group. The protecting groups are not limited to the specific groups as mentioned above.

These protecting groups may have an alkyl group, a halogen atom, a nitro group, or an alkoxyl group as a substituent, when the protecting groups include a phenyl group.

A cation of Compound [IV] may be a metal cation such as sodium, potassium and lithium, and an ammonium cation such as tetraethylammonium. Sodium ion is preferably used.

The salt of formula [IV] can be prepared from adenine of formula [V]: 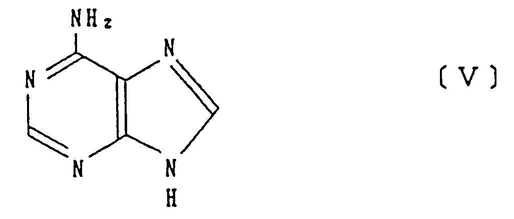
eg by reaction with a base providing the cation X⁺. Thus this sodium salt can be obtained by the reaction of Compound [V] with sodium hydroxide or a sodium alcoholate in water or alcohol at room temperature usually within 30 minutes. The sodium salt is stable and easy to handle.

Condensation of Compound [IV] with Compound [III] is carried out in a solvent having moderate polarity such as acetone, acetonitrile, 1,2-dichloroethane, dichloromethane, diethylether, 1,2-methoxyethane, tetrahydrofuran and ethyl acetate, at room temperature, and it is usually completed with 19 hours. Acetone is preferably used.

When the reaction mixture contains trace amounts of position isomers, the mixture of α- and β-isomers produced may be isolated from the reaction mixture by an appropriate means such as column chromatography, and then purified, e.g. by recrystallization, to obtain the pure β-isomer of compound [II], and protecting groups may then be eliminated by hydrolysis, alcoholysis, ammonolysis or the like to obtain 2′-deoxy-β-adenosine [I].

As mentioned above, 2′-deoxy-β-adenosine can be obtained in a high yield by the process of the present invention without using expensive or dangerous reagents.

The present invention will be illustrated by the following examples, but the present invention is not restricted thereto.

### (1) Sodium salt of adenine

0.54 g (10 mmol) of sodium methylate was added to a suspension of 1.4 g (10 mmol) of adenine [V] in 50 ml of methanol, and the mixture was stirred at room temperatures for 30 minutes.

After the crystals dissolved completely, the solvent was distilled away under reduced pressure, and the white crystals obtained were dried under reduced pressure to obtain 1.6 g of the captioned compound. Yield 100 %
¹H-NMR(D₂O): δ 8.03(s, 1H), 7.96(s, 1H)

### (2) 9-(2-deoxy-3,5-di-O-p-toluoyl-β-D-erythro-pentofuranosyl)adenine

0.45 g (1.3 mmol) of dry-powdered 1-chloro-2-deoxy-3,5-di-O-p-toluoyl-β-D-erythro-pentofuranose was added to a suspension of 0.40 g (2.5 mmol) of sodium salt of adenine in 25 ml of absolute acetone, and the mixture was stirred at room temperatures for 19 hours.

After the reaction was completed, the reaction solution was poured into an aqueous solution of sodium chloride, and the reaction mixture was extracted with methylene chloride. The organic layer was dried over anhydrous magnesium sulfate, and then the solvent was distillated away under reduced pressure.

The residue obtained was purified by silicagel column chromatography with a mixture of methylene chloride and acetone (volume ratio of methylene chloride to acetone = 50:50) as a solvent.

The captioned compound was crystallized from ethyl acetate, to obtain 0.29 g of the captioned compound, which was crystals having 1/2 molecule of ethyl acetate. Yield 43 %.
m.p. : 1 6 8 - 1 6 9 °C
¹H-NMR(CDCℓ₃):δ 8.33(s,1H,H-8),7.98(s,1H,H-2),
7.97(d,J=9.3Hz,2H,aromatic-H),7.91(d,J=8.2Hz,2H,aromatic-H),
7.28(d,J=8.8Hz,2H,aromatic-H),7.22(d,J=8.2Hz,2H,aromatic-H),
6.54(dd,J=8.4&5.8Hz,1H,H-1'),5.81(dt,J=6.4&2.2Hz,1H,H-3'),
5.73(br.,2H,NH₂),4.76(dd,J=11.8&3.8Hz,1H,H-5'),
4.67(dd,J=11.8&4.4Hz,1H,H-5''),4.64(m,1H,H-4'),
3.11(ddd,J=14.3&8.2&6.2Hz,1H,H-2'),
2.83(ddd,J= 14.2&5.8&2.0Hz,1H,H-2''),
2.44(s,3H,CH₃), 2.40(s,3H,CH₃)

### (3) 2'-deoxy-β-adenosine [I]

20 ml of ammonia water was added to a solution of 0.97 g (1.8 mmol) of 9-(2-deoxy-3,5-di-O-p-toluoyl-β-D-erythro-pentofuranosyl)adenine in 100 ml of methanol, and the mixture was stirred at room temperatures for 24 hours.

After the reaction was completed, the reaction solution was concentrated under reduced pressure, and the residue obtained was dissolved in water and washed twice with chloroform.

The aqueous layer was concentrated under reduced pressure, and the crystals obtained were recrystallized from water to obtain 0.44 g of monohydrate of the captioned compound. Yield 89 %.
m.p. : 1 8 9 - 1 9 0 °C
(m.p. disclosed in literatures : 1 8 7 - 1 8 9 °C)
¹H-NMR(CD₃OD):δ 8.31(s,1H,H-8),8.18(s,1H,H-2),
6.43(dd,J=8.0&5.9Hz,1H,H-1'),4.58(dt,J=5.8&2.7Hz,1H,H-3'),
4.07(q,J=2.9Hz,1H,H-4'),3.85(dd,J=12.4&2.9Hz,1H,H-5'),
3.75(dd,J=12.3&3.4Hz,1H,H-5''),
2.81(ddd,J=13.5&7.9&5.7Hz,1H,H-2'),
2.41(ddd,J=13.4&6.0&2.8Hz,1H,H-2'')

## Claims

1. A process for preparing 2′-deoxy-β-adenosine having the formula [I]: 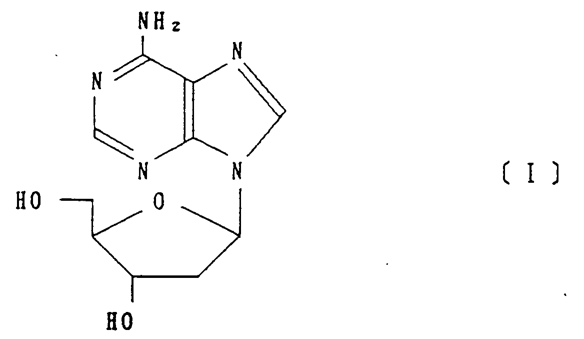 characterized in that a salt of adenine having the general formula [IV]: 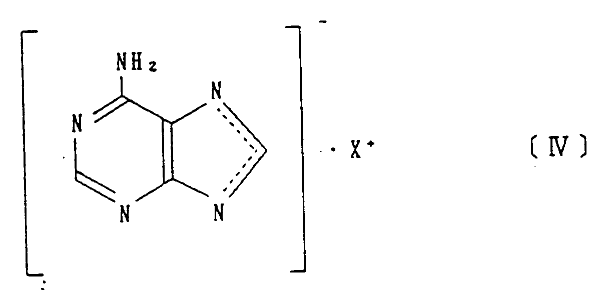 (wherein X⁺ is a cation)
is allowed to condense with a protected derivative of 1-chloro-2-deoxy-α-D-erythro-pentofuranose having the general formula [III]: 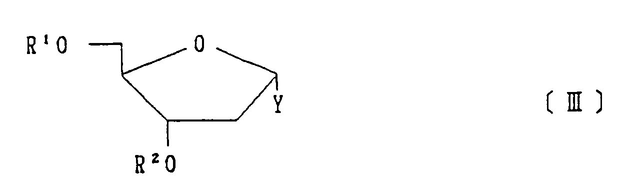 (wherein R¹ and R² are independently hydroxyl protecting groups and Y is a halogen)
to produce a derivative of 9-(2-deoxy-β-D-erythro-pentofuranosyl) adenine having the general formula [II]: 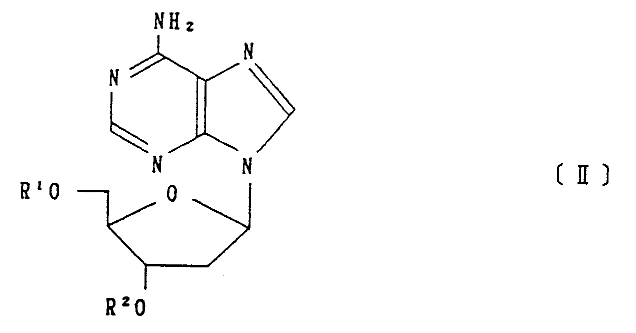 (wherein R¹ and R² are as above defined) and then the protecting groups are eliminated.

2. A process as claimed in claim 1 in which the protecting groups R¹ and R² are aralkyl, acyl, alkoxycarbonyl or aryloxycarbonyl groups.

3. A process as claimed in claim 1 or claim 2 in which the cation X⁺ is a sodium, potassium, lithium or tetraethylammonium cation.

4. A process as claimed in any one of claims 1 to 3 in which the condensation is effected in a solvent of moderate polarity.

## Patentansprüche

1. Verfahren zur Herstellung von 2'-Desoxy-β-adenosin mit der Formel [I]: 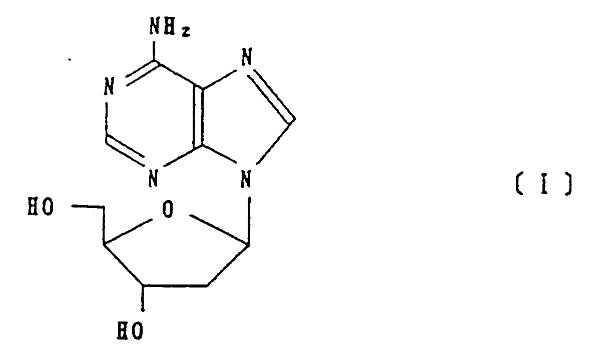 **dadurch gekennzeichnet,**
daß man ein Adeninsalz mit der allgemeinen Formel [IV]: 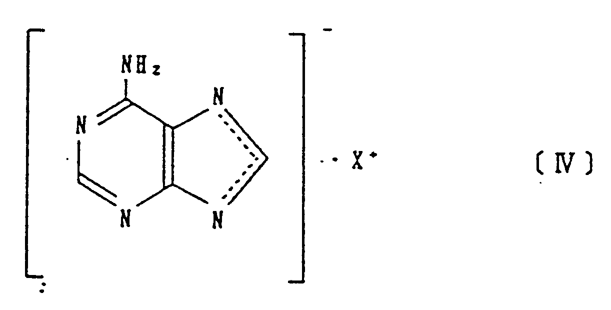 (wobei X⁺ ein Kation ist)
mit einem geschützten Derivat von 1-Chlor-2-desoxy-α-D-erythro-pentofuranose mit der allgemeinen Formel [III] kondensieren läßt: 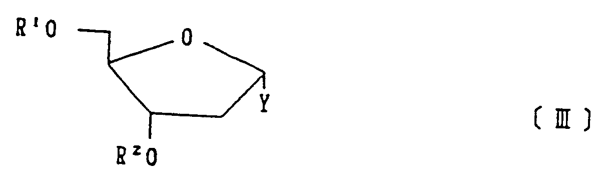 (wobei R¹ und R² unabhängig Hydroxylschutzgruppen sind und Y ein Halogen ist)
um ein Derivat von 9-(2-Desoxy-β-D-erythropento-furanosyl)adenin mit der allgemeinen Formel [II] herzustellen: 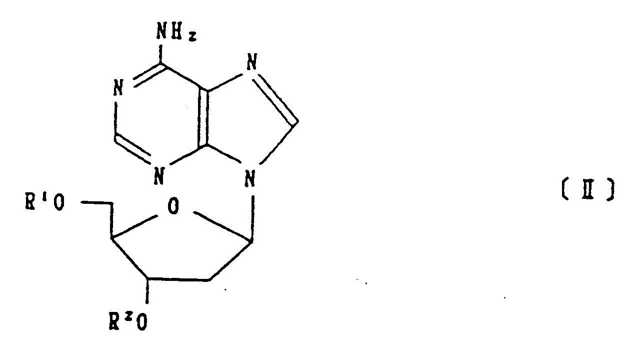 (wobei R¹ und R² wie oben definiert sind),
und anschließend werden die Schutzgruppen eliminiert.

2. Verfahren nach Anspruch 1, wobei die Schutzgruppen R¹ und R² Aralkyl-, Acyl-, Alkoxycarbonyl- oder Aryloxycarbonylgruppen sind.

3. Verfahren nach Anspruch 1 oder 2, wobei das Kation X⁺ ein Natrium-, Kalium-, Lithium- oder Tetraethylammoniumkation ist.

4. Verfahren nach einem der Ansprüche 1 - 3, wobei die Kondensation in einem Lösungsmittel mit gemäßigter Polarität durchgeführt wird.

## Revendications

1. Procédé de préparation de 2'-désoxy-β-adénosine ayant la formule (I): 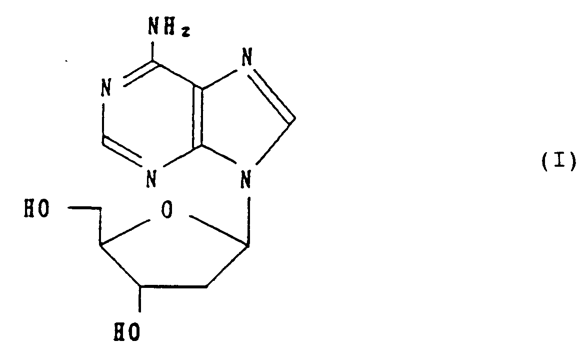 caractérisé en ce qu'un sel d'adénine ayant la formule générale (IV): 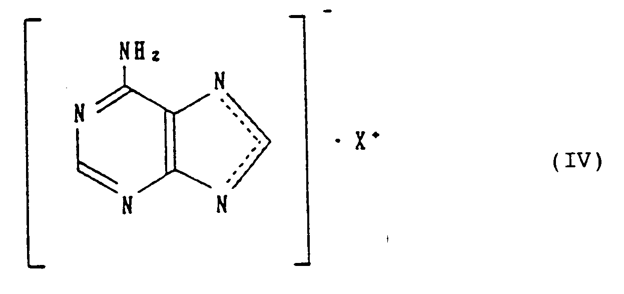 (dans laquelle X⁺ est un cation)
est soumis à une réaction de condensation avec un dérivé protégé de 1-chloro-2-désoxy-α-D-érythro-pentofuranose ayant la formule générale (III): 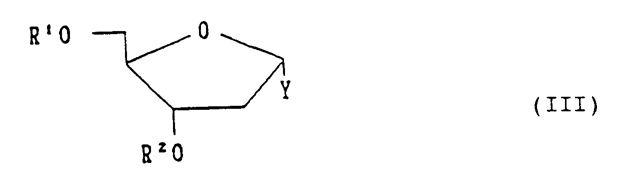 (dans laquelle R¹ et R² sont indépendamment des groupes protecteurs d'hydroxyle et Y est un halogène),
pour donner un dérivé de 9-(2-désoxy-β-D-érythro-pentofuranosyl)adénine ayant la formule générale (II): 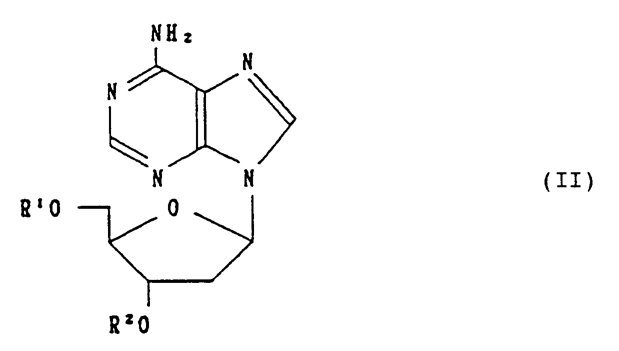 (dans laquelle R¹ et R² sont tels que définis ci-dessus)
après quoi les groupes protecteurs sont éliminés.

2. Procédé selon la revendication 1, dans lequel les groupes protecteurs R¹ et R² sont des groupes aralkyle, acyle, alkoxycarbonyle ou aryloxycarbonyle.

3. Procédé selon la revendication 1 ou la revendication 2, dans lequel le cation X⁺ est un cation sodium, potassium, lithium ou tétraéthylammonium.

4. Procédé selon l'une quelconque des revendications 1 à 3, dans lequel la condensation est effectuée dans un solvant de polarité moyenne.
